(19) Europäisches Patentamt
European Patent Office
Office européen des brevets

(11) Veröffentlichungsnummer: **0 272 605**
**A2**

# (12) EUROPÄISCHE PATENTANMELDUNG

(21) Anmeldenummer: **87118669.8**

(22) Anmeldetag: **16.12.87**

(51) Int. Cl.⁴: **C12P 41/00**, C12P 17/10, C12P 7/42

Der (Die) Mikroorganismus (Mikroorganismen) ist (sind) bei Deutsche Sammlung von Microorganismen unter der (den) Nummer(n) 3906 and 3907 hinterlegt worden.

(30) Priorität: **23.12.86 CH 5151/86**

(43) Veröffentlichungstag der Anmeldung: **29.06.88 Patentblatt 88/26**

(84) Benannte Vertragsstaaten: **AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Leuenberger, Hans Georg, Dr.**
**Rebenstrasse 23**
**CH-4112 Bättwil(CH)**
Erfinder: **Matzinger, Peter Karl, Dr.**
**Buchenstrasse 2**
**CH-4118 Rodersdorf(CH)**
Erfinder: **Wirz, Beat, Dr.**
**Wiedenweg 4**
**CH-4153 Reinach(CH)**

(74) Vertreter: **Bertschinger, Christoph, Dr. et al**
**Grenzacherstrasse 124 P.O. Box 3255**
**CH-4002 Basel(CH)**

## (54) Verfahren zur Herstellung von optisch aktiven 2-Hydroxybuttersäurederivaten.

(57) Durch fermentative Reduktion von Verbindungen der Formel

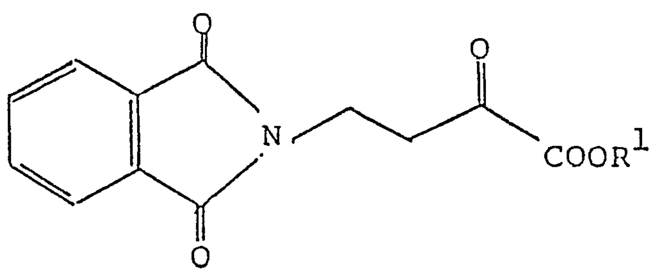

II

worin R¹ Wasserstoff oder einen üblichen Esterrest bedeutet, erhält man optisch aktive Hydroxybuttersäurederivate der Formel

EP 0 272 605 A2

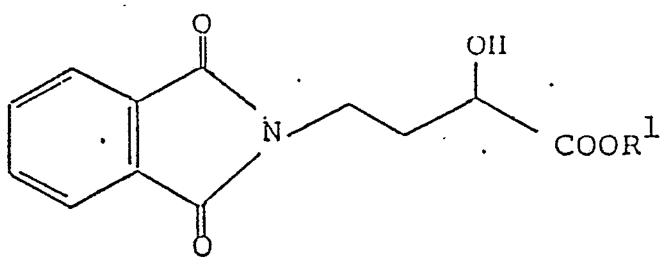

I

worin $R^1$ obige Bedeutung besitzt.

Die Verbindungen der Formel I sind wertvolle Zwischenprodukte, insbesondere bei der Herstellung von optisch aktivem 3-Hydroxy-2-pyrrolidinon und von Derivaten davon.

## Verfahren zur Herstellung von optisch aktiven 2-Hydroxybuttersäurederivaten

Die Erfindung betrifft ein mikrobiologisches Verfahren zur Herstellung von optisch aktiven 2-Hydroxybuttersäurederivaten. Die erfindungsgemäss erhältlichen Verbindungen sind wertvolle Zwischenprodukte in organischen Synthesen. So können sie bei der Herstellung von optisch aktivem 3-Hydroxy-2-pyrrolidinon und von Derivaten davon verwendet werden, beispielsweise zur Herstellung der in EP-A-24030, 57846 und 71216 beschriebenen optisch aktiven 3-Hydroxy-2-pyrrolidinon-Derivate, welche sich bei geringer Toxizität durch wertvolle pharmakodynamische Eigenschaften auszeichnen und zur Bekämpfung bzw. Verhütung der cerebralen Insuffizienz bzw. zur Verbesserung der intellektuellen Leistungsfähigkeit verwendet werden können.

Das erfindungsgemässe Verfahren ermöglicht die Herstellung von optisch aktiven 2-Hydroxybuttersäurederivaten der allgemeinen Formel

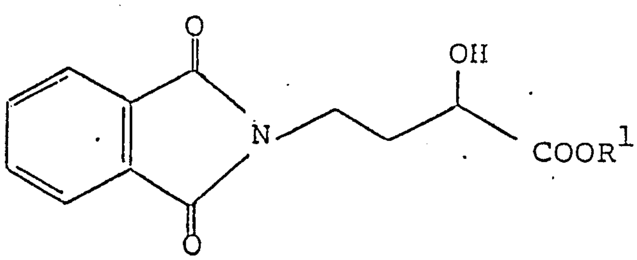

I

worin $R^1$ Wasserstoff oder einen üblichen Esterrest bedeutet;
es ist dadurch gekennzeichnet, dass man eine Verbindung der allgemeinen Formel

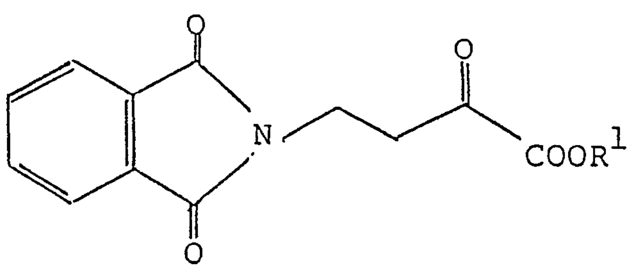

II

worin $R^1$ obige Bedeutung besitzt,
fermentativ reduziert.

Der Ausdruck "üblicher Esterrest" umfasst die in Carbonsäureestern üblicherweise vorkommenden Reste, wie Alkyl, Aryl, Aralkyl, Alkylaryl und dergleichen, beispielsweise Methyl, Aethyl, Isopropyl, t-Butyl, Phenyl, Benzyl und Tolyl. Vorzugsweise bedeutet $R^1$ in den obigen Formeln I und II $C_1$-$C_6$-Alkyl, insbesondere Aethyl oder Isopropyl.

Das erfindungsgemässe Verfahren eröffnet einen neuen vorteilhaften Weg zur Herstellung optisch aktiver Verbindungen der Formel I.

Das erfindungsgemässe Verfahren wird zweckmässig in der Weise durchgeführt, dass ein die Verbindungen der Formel I erzeugender Mikroorganismus in einem wässerigen Nährmedium in Anwesenheit einer Verbindung der Formel II gezüchtet wird, bzw. nach der Anzucht in Anwesenheit einer Verbindung der Formel II weiter inkubiert wird.

Je nach verwendetem Mikroorganismus wird dabei vorwiegend die (R)-oder (S)-Form der Verbindungen der Formel I gebildet. Geeignete, die Verbindungen der Formel I erzeugende Mikroorganismen können leicht durch Austesten von beliebigen Mikroorganismen - vorzugsweise aeroben Hefen - auf das erfindungsgemäss eingesetzte Ausgangsprodukt der Formel II gefunden werden.

Nachstehend werden Beispiele bevorzugt verwendbarer Mikroorganismen aufgeführt, jeweils mit Angabe der Stereochemie der vorwiegend gebildeten Verbindung der Formel I:

1. Prokaryonten

1.1. Mycelbildende Bakterien (Actinomyceten) der Gattung: Actinoplanes
wie Actinoplanes sp. (z.B. ATCC 31041): R
Rhodococcus
wie Rhodococcus rhodochrous (z.B. ATCC 4273): R

2. Eukaryonten

2.1. Pilze der GattungBeauveria
wie Beauveria bassiana (z.B. ATCC 13144): R
Byssochlamys
wie Byssochlamys fulva (z.B. NRRL 1125): R
Choanephora
wie Choanephora circinans (z.B. NRRL 2546): S
Glomerella
wie Glomerella rubicola (z.B. CBS 200.35): R

2.2. Hefen der GattungCandida
wie Candida albicans (z.B. ATCC e10231): R

Candida parapsilosis (z.B. NRRL Y-2315; DSM 3907): R
Candida rugosa (z.B. NRRL Y-1496): R
Candida sorbophila (z.B. CBS 7072): R
Candida tropicalis (z.B. ATCC 9968): R
Candida utilis (z.B. CBS 621): R
Hansenula
wie Hansenula anomala (z.B. ETH 317): S
Rhodosporidium
wie Rhodosporidium toruloides (z.B. NRRL Y-1091): S
Rhodotorula
wie Rhodotorula minuta (z.B. NRRL Y-1589): R
Saccharomyces
wie Saccharomyces cerevisiae (z.B. NRRL Y-567): S
Torulopsis
wie Torulopsis magnoliae (z.B. NRRL Y-2024; DSM 3906): S
Torulopsis rotundata (z.B. NRRL Y-1402): R

Die obigen Angaben in Klammern beziehen sich jeweils auf entsprechende Stämme, die bei einer der folgenden Hinterlegungsstellen unter der angegebenen Nummer hinterlegt sind:
NRRL = Northern Utilization Research and Development Division of U.S.D.A., Peoria, Illinois, USA
ATCC = American Type Culture Collection, Rockville, Maryland, USA
CBS = Centraal-Bureau voor Schimmelcultures, Baarn, Holland
ETH = Eidgenössische Technische Hochschule, Zürich, Schweiz
DSM = Deutsche Sammlung von Mikroorganismen, Göttigen, Bundesrepublik Deutschland

Vorzugsweise werden Mikroorganismen verwendet, welche das gewünschte Produkt der Formel I in einer optischen Reinheit von mindestens 90% ee liefern. Bevorzugt verwendbare Mikroorganismen im erfindungsgemässen Verfahren sind die Hefen, insbesondere der Gattungen Candida und Torulopsis. Besonders bevorzugt sind Mikroorganismen der Species Candida parapsilosis und Torulopsis magnoliae, insbesondere die oben angegebenen hinterlegten Stämme und Mutanten davon. Zur Sicherstellung der Durchführbarkeit des Verfahrens wurden Subkulturen der Hefen Candida parapsilosis NRRL Y-2315 und Torulopsis magnoliae NRRL Y-2024 am 4. Dezember 1986 bei DSM unter den Nummern 3907 bzw. 3906 neu hinterlegt.

Es versteht sich, dass jeder erfindungsgemäss eingesetzten Mikroorganismen vor der Verwendung in der erfindungsgemässen Fermentation angezüchtet werden soll. Die Anzucht erfolgt in der Regel in an sich bekannter Weise in einem wässerigen Medium unter Zuhilfenahme der üblichen Nährstoffe, d.h. in Gegenwart einer Kohlenstoffquelle (z.B. Glucose, Fructose, Saccharose, Maltose und/oder Malzextrakt) und

einer Stickstoffquelle (z.B. Harnstoff, Pepton, Hefeextrakt, Fleischextrakt, Aminosäuren und/oder Ammoniumsalze).

Das Anzuchtsmedium mit dem darin gewachsenen Mikroorganismus kann in der Regel direkt für die erfindungsgemässe Reduktion verwendet werden, die dann nach Zugabe des Ausgangsprodukts der Formel II ohne weitere Zusätze durchführbar ist.

Die Zusammensetzung des erfindungsgemäss verwendeten Reaktionsmediums kann aber auch wesentlich einfacher sein und z.B. lediglich aus einer Lösung des Ausgangsprodukts der Formel II und dem separat angezüchteten Mikroorganismus bestehen. Es ist jedoch vorteilhaft, dem wässrigen Medium eine assimilierbare Kohlenstoffquelle (beispielsweise in Form eines Zuckers, wie Glucose, Fructose, Saccharose, Maltose und dergleichen) als Mikroorganismennährstoff zuzusetzen, damit die Lebensfähigkeit und die damit verbundene Stoffwechselaktivität des Mikroorganismus möglichst lange erhalten bleiben. Vorzugsweise wird die Kohlenstoffquelle in einer Menge von etwa 10-100 g/l zugesetzt. Bei langen Fermentationszeiten ist es von Vorteil, die Kohlenstoffquelle in einer bevorzugten Menge von 10-100 g/l einmal oder mehrmals nachzufüttern.

Der Zusatz einer Stickstoffquelle zum Reaktionsmedium ist nicht notwendig. Gewünschtenfalls kann aber eine assimilierbare Stickstoffquelle (beispielsweise in Form von Harnstoff, Pepton, Hefeextrakt, Fleischextrakt, Aminosäuren, Ammoniumsalzen und dergleichen) zugesetzt werden, vorzugsweise in einer Menge von etwa 1-50 g/l. Das Fermentationsmedium kann ferner auch anorganische Salze, wie Magnesium-, Natrium-, Kalium-und/oder Ferrosalze, und andere wachstumsfördernde Substanzen, wie Vitamine und dergleichen, enthalten.

Der pH-Wert des Reaktionsmediums soll vorzugsweise innerhalb des Bereiches von 2 bis 10, insbesondere 3 bis 8 liegen, ein Bereich, der zumeist ohne besondere Zusätze erreichbar ist. Gewünschtenfalls kann der pH-Wert durch Verwendung von Puffern, Säuren oder Basen, z.B. Phosphat-, Phthalat-oder Trispuffern [Tris-(Hydroxymethyl)-aminomethan], Salzsäure, Natronlauge und dergleichen, eingestellt werden. Die Temperatur kann in weiten Grenzen schwanken, z.B. zwischen 10°C und 40°C, vorzugsweise zwischen 20°C bis 35°C.

Um optimale Ausbeuten zu erhalten, ist es vorteilhaft, das Ausgangsprodukt der Formel II in der Gärbrühe in einer Konzentration von etwa 0,05-5,0 Gew.-%, insbesondere etwa 0,1-1,0 Gew.-%, einzusetzen. Nach erfolgter Reaktion kann erneut Ausgangsprodukt in einer bevorzugten Konzentration von etwa 0,1-1,0 Gew.-% zugesetzt werden. Dieses Verfahren lässt sich bis zur Inaktivierung der Mikroorganismen wiederholen. Das Substrat kann auch mit Hilfe einer Pumpe kontinuierlich zugefügt werden.

Die nützliche Fermentationszeit ist von den verwendeten Mikroorganismen abhängig. Sie liegt bei einmaliger Zugabe des Ausgangsprodukts im allgemeinen zwischen etwa 2 und 100 Stunden, insbesondere zwischen etwa 4 und 24 Stunden. Häufig genügen aber auch kürzere Fermentationszeiten. Bei mehrmaliger oder kontinuierlicher Zugabe des Ausgangsprodukts kann sich die Fermentationszeit entsprechend verlängern.

Die erfindungsgemässe Reduktion wird vorzugsweise aerob durchgeführt, z.B. unter Rühren, Schütteln unter Luftzutritt oder mittels einer Belüftungsvorrichtung. Zur Schaumbekämpfung können die üblichen Antischaummittel, wie Siliconöle, Polyalkylenglykol-Derivate, Sojabohnenöl und dergleichen, zugesetzt werden. Vorzugsweise verwendet man einen Mikroorganismus, dessen Wachstum im Anzuchtsmedium abgeschlossen ist (stationäre Phase). Die Wahl eines stationären Mikroorganismus hat den Vorteil, dass die Biotransformation nicht unter sterilen Bedingungen ausgeführt werden muss, sofern die Reduktion in einem Medium stattfindet, das keine weitere Vermehrung von Fremd-Mikroorganismen zulässt, beispielsweise ein Reaktionsmedium ohne oder mit erschöpfter Stickstoffquelle.

Nach Beendigung der Reaktion kann das Fermentationsprodukt in üblicher Weise aus der Gärbrühe isoliert werden. Vorzugsweise kommt eine Extraktion mit einem nicht wasserlöslichen organischen Lösungsmittel in Betracht, beispielsweise mit einem aliphatischen oder cycloaliphatischen, gegebenenfalls chlorierten Kohlenwasserstoff, einem aliphatischen Ester oder einem aliphatischen Aether, wie Hexan, Cyclohexan, Methylenchlorid, Chloroform, Tetrachlorkohlenstoff, Aethylacetat, Butylacetat, Amylacetat, Diäthyläther, Diisopropyläther und dergleichen. Ein bevorzugtes Lösungsmittel ist Aethylacetat.

Das erhaltene Rohprodukt kann gewünschtenfalls in üblicher Weise, z.B. durch Destillation, Kristallisation, chromatographische Methoden etc., gereinigt werden.

Die Ausgangsprodukte der Formel II können nach an sich bekannten Methoden hergestellt werden, zweckmässigerweise wie folgt:

3-Brombutyrolacton wird in 3-Hydroxybutyrolacton übergeführt, beispielsweise mittels Kaliumcarbonat in Wasser, und hieraus wird dann α-Hydroxy-1,3-dioxo-2-isoindolbuttersäure hergestellt, z.B. durch Umsetzung mit Phthalimid-Kalium in Dimethylformamid. Diese Säure oder ein daraus auf übliche Weise (z.B. mittels eines geeigneten Alkohols in Gegenwart von Säure) hergestellter Ester wird dann zur entsprechenden α-1,3-Trioxoverbindung der Formel II oxidiert, beispielsweise mittels Dimethylsulfoxid und Acetanhydrid oder mittels eines katalytischen Oxidationssystems, zweckmässigerweise mit einem sauerstoffhaltigen Gas in Gegenwart von Vanadylacetylacetonat als Katalysator und Toluol als Lösungsmittel.

Da die Verbindungen der Formel II eine prochirale Struktur besitzen, sind erfindungsgemäss, unter Verwendung verschiedener Mikroorganismen, beide Enantiomeren der Verbindungen der Formel I zugänglich.

Aus den erfindungsgemäss erhaltenen Verbindungen der Formel I kann man durch Spaltung der Phthalimidogruppe und einer allfälligen Estergruppe (R)-bzw. (S)-4-Amino-2-hydroxybuttersäure erhalten (zweckmässigerweise durch saure Hydrolyse, z.B. mittels Salzsäure), und diese kann dann beispielsweise gemäss den Angaben in EP-A-24030, 57846 und 71216 zu therapeutisch wertvollen optisch aktiven 3-Hydroxy-2-pyrrolidinon-Derivaten weiterverarbeitet werden.

Das erfindungsgemässe Verfahren wird durch die folgenden Beispiele 3-6 weiter veranschaulicht. Die Ausgangsprodukte werden jeweils in Pulverform, gelöst in Aethylacetat oder in einer Suspension eingesetzt. Zur Bestimmung der optischen Reinheit kann man die erhaltenen Produkte mit (-)-Camphansäurechlorid derivatisieren und das Enantiomerenverhältnis gaschromatographisch (Diastereomere) bestimmen; die optische Reinheit wird als "enantiomeric excess" (ee) ausgedrückt.

Beispiele 1 und 2 illustrieren die Herstellung von Ausgangsprodukten der Formel II, und Beispiel 7 illustriert die Herstellung von optisch aktiver 4-Amino-2-hydroxybuttersäure aus den erfindungsgemäss erhältlichen Verbindungen der Formel I. In allen diesen Beispielen sind sämtliche Temperaturen in Celsiusgraden angegeben.

<u>Beispiel 1</u>

a) Unter Rühren werden zu einer Lösung von 2,04 kg (14,7 Mol) Kaliumcarbonat in 9,6 l Wasser innerhalb von ca. 2 Stunden bei 85-90° vorsichtig 1,6 kg (9,7 Mol) 3-Brombutyrolacton zugetropft, wonach noch 1/2 Stunde bei 85° nachgerührt wird. Anschliessend wird das Reaktionsgemisch bei 50° langsam mit 1,6 l (ca. 19 Mol) 37%iger Salzsäure auf pH 1 angesäuert (wobei eine heftige $CO_2$-Entwicklung erfolgt) und im Vakuum zu einem kristallinen Brei eingedampft. Dieser Kristallbrei wird in 5 l Aceton unter Rühren suspendiert, worauf man filtriert und den Filterrückstand mit dreimal 1,5 l Aceton nachwäscht. Das braune Filtrat wird unter Rühren 1 Stunde mit 1 kg Natriumsulfat getrocknet. Danach filtriert man erneut, wäscht das Natriumsulfat mit 2 l Aceton nach und dampft das Filtrat im Vakuum bei ca. 50° ein. Man erhält rohes 3-Hydroxybutyrolacton, welches in einer Destillationsapparatur wie folgt gereinigt wird:

Zunächst wird am Wasserstrahlvakuum bei 80° Badtemperatur Leichtflüchtiges abgetrennt, und danach wird am Hoch vakuum bei 120-140° Badtemperatur das Hauptprodukt destilliert. Man erhält bei 92-100° 0,3-0,1 mm 3-Hydroxybutyrolacton mit einem Gehalt von 95%.

b) Unter Rühren wird ein Gemisch, bestehend aus 3,2 kg (17,3 Mol) Phthalimid-Kalium, 1,85 kg (18,1 Mol) ca. 95%igem 3-Hydroxy-butyrolacton und 4 l Dimethylformamid, während 9 Stunden am Rückfluss gekocht (Badtemperatur ca. 180°); während der Reaktion entsteht aus dem heterogenen Gemisch eine Lösung, aus welcher sich langsam wieder ein Kristallisat abscheidet. Anschliessend werden zum auf Raumtemperatur abgekühlten Reaktionsgemisch zunächst 9,5 l Wasser zugegeben (wobei eine dunkle Lösung mit pH 5-6 entsteht) und danach innerhalb 1/2 Stunde 1,55 l (ca. 18,5 Mol) 37%ige Salzsäure zugetropft, wobei ein Niederschlag ausfällt. Während 1 Stunde wird bei 0-5° nachgerührt. Danach wird die Suspension filtriert, und der Filterrückstand wird portionenweise mit 15 l Eiswasser nachgewaschen. Nach Trocknen im Vakuumtrockenschrank bei 80° erhält man rohe α-Hydroxy-1,3-dioxo-2-isoindolbuttersäure, Smp. 136-146° (sint.).

Zur Reinigung werden unter Rühren 0,5 kg rohe α-Hydroxy-1,3-dioxo-2-isoindolbuttersäure mit 50 g Aktivkohle und 50 g Kieselgur in 8 l Essigester während 1/4 Stunde am Rückfluss gekocht. Das heisse Gemisch wird im Teilvakuum über eine Glassinternutsche filtriert, und der Filterrückstand wird mit 1 l Essigester von 75° nachgewaschen. Das Filtrat wird am Teilvakuum bei 60-70° auf ein Volumen von ca. 2 l eingeengt, wobei das Produkt auskristallisiert. Die Kristallisation wird während 3 Stunden unter Rühren bei 0° vervollständigt. Durch Filtration, Nachwaschen mit 0,5 l Essigester von 0° und Trocknen im Vakuumtrockenschrank erhält man α-Hydroxy-1,3-dioxo-2-isoindolbuttersäure vom Smp. 149-151°.

c) Eine Suspension von 1,0 kg (4,0 Mol) α-Hydroxy-1,3-dioxo-2-isoindolbuttersäure in 4 l Isopropanol wird unter gutem Rühren innerhalb von 10 Minuten mit 40 ml 96%iger Schwefelsäure versetzt und anschliessend während 7 Stunden am Rückfluss gekocht. Nach Abkühlen des Reaktionsgemisches auf Raumtemperatur versetzt man mit 15 l Wasser, gibt zur Neutralisation allmählich insgesamt 4 l gesättigte Natriumbicarbonatlösung zu und extrahiert dann mit viermal je 5 l tert.Butylmethyläther. Die organischen

Phasen werden über 1 kg Natriumsulfat getrocknet und nach Filtration am Rotationsverdampfer bei 40° 20 mbar bis zur Gewichtskonstanz eingedampft. Man erhält rohes Isopropyl-α-hydroxy-1,3-dioxo-2-isoindolbutyrat in Form eines gelblichen Oels, welches bei Raumtemperatur kristallisiert.

Zur Umkristallisation löst man das erhaltene Rohprodukt in 5 l siedendem Isopropyläther; nach Abkühlen rührt man die Lösung zunächst einige Stunden ohne Kühlung (wobei bald Kristallisation einsetzt) und dann 2 Stunden im Eisbad. Das Produkt wird abfiltriert, mit 0,5 l Isopropyläther von 0° gewaschen und bei 30° l mbar bis zur Gewichtskonstanz getrocknet. Man erhält Isopropyl-α-hydroxy-1,3-dioxo-2-isoindolbutyrat in Form farbloser Kristalle vom Smp. 76-78°.

Aus der Mutterlauge erhält man in analoger Weise noch eine weitere Portion dieses Produkts vom Smp. 75-77°.

In analoger Weise kann auch der entsprechende Aethylester hergestellt werden; Smp. 90-91°.

d) Eine Lösung von 99 g (0.34 Mol) Isopropyl-α-hydroxy-1,3-dioxo-2-isoindolbutyrat in 1,07 l Dimethylsulfoxid wird unter Rühren auf 18° gekühlt; nach Entfernen des Kühlbades werden innerhalb von 25 Minuten 136 ml Acetanhydrid (147 g bzw. 1,44 Mol) zugetropft, wobei die Temperatur auf 13° sinkt. Man erwärmt auf 19° und rührt 17 Stunden bei Raumtemperatur nach.

Hierauf versetzt man mit 7 l Eiswasser und dann mit 1 l gesättigter Natriumbicarbonatlösung, extrahiert dreimal mit je 3 l tert.-Butylmethyläther, trocknet die vereinigten Extrakte über 0,5 kg Natriumsulfat, filtriert und dampft am Rotationsverdampfer bei 40° 20 mbar bis zur Gewichtskonstanz ein. Man erhält rohes Isopropyl-α-1,3-trioxo-2-isoindolbutyrat in Form eines gelbes Oels.

Zur Kristallisation löst man das erhaltene Rohprodukt in 200 ml Essigester, setzt zunächst 250 ml Hexan zu und rührt bis zur deutlich einsetzenden Kristallisation; dann werden weitere 430 ml Hexan hinzugefügt. Die Suspension wird über Nacht bei Raumtemperatur und 1 Stunde im Eisbad gerührt und dann filtriert; der Filterrückstand wird mit 100 ml Hexan/Essigester 4:1 gewaschen und bei 30° 20 mbar bis zur Gewichtskonstanz getrocknet. Man erhält Isopropyl-α-1,3-trioxo-2-isoindolbutyrat in Form farbloser Kristalle vom Smp. 98,5-100°.

In analoger Weise kann auch der entsprechende Aethylester hergestellt werden; Smp. 82-84°.

Beispiel 2

Unter Rühren und Erwärmen auf 90° werden 100 g (0,343 Mol) Isopropyl-α-hydroxy-1,3-dioxo-2-isoindolbutyrat in 350 ml Toluol gelöst. Zur klaren Lösung werden, noch während der Aufwärmephase, 3,4 g Vanadyl-acetylacetonat (12,8 mMol) als Katalysator gegeben. Sobald der Katalysator gelöst ist, werden noch 3,4 ml Pyridin zugegeben. Nun werden unter starkem Rühren bei 90° 1 l/Min. 8% Sauerstoff durch eine Gaseinleitungskerze eingeleitet. Die Reaktion, die dünnschichtchromatographisch verfolgt werden kann, ist nach ca. 3 Stunden beendet. Das Volumen des abgeschiedenen Wassers beträgt 4-5 ml.

Das dunkle Rektionsgemisch wird durch ein Polster aus Kieselgur filtriert und am Rotationsverdampfer (Badtempera tur 50°) im Wasserstrahlvakuum weitgehend eingeengt. Der Rückstand wird in 50-100 ml Toluol/Essigester 2:1 aufgenommen und zur Abtrennung des löslichen Katalysatorteils über 200 g Kieselgel filtriert. Das Produkt wird mit ca. 1 l Toluol/Essigester 2:1 eluiert und in Fraktionen à 300 ml gesammelt. Die das gewünschte Produkt enthaltenden Fraktionen werden vereinigt und am Rotationsverdampfer (Bad 50°) im Wasserstrahlvakuum eingedampft. Der braun-gelbe Eindampfrückstand wird in 65 ml siedendem Essigester gelöst, worauf die Lösung in der Wärme mit 100 ml Hexan versetzt wird. Beim Abkühlen auf Raumtemperatur erfolgt Kristallisation, welche bei 5° (Kühlschrank) über Nacht vervollständigt wird. Die Kristalle werden abfiltriert, mit ca. 300 ml vorgekühltem (-15°) Hexan/Essigester 3:1 sowie mit ca. 50 ml Hexan gewaschen und anschliessend bis zur Gewichtskonstanz bei 40° im Wasserstrahlvakuum getrocknet. Man erhält Isopropyl-α-1,3-trioxo-2-isoindolbutyrat in Form weissbeiger Kristalle vom Smp. 95,5-98,5°.

Durch Umkristallisation aus Toluol kann man hieraus weisse Kristalle vom Smp. 98-100° erhalten.

Beispiel 3

Die Hefe Candida parapsilosis NRRL Y-2315 wird auf zwei Schrägagars mit Medium 1 (bestehend aus 1 Gew.-% Hefeextrakt, 1 Gew.-% Pepton, 2 Gew.-% Glucose, 1,6 Gew.-% Agar und 94,4 Gew.-% destilliertem Wasser) gezüchtet. Nach 3 Tagen Inkubation bei 30° versetzt man die Ansätze mit je 9 ml 0,9%iger (Gew./Vol.) Kochsalzlösung und suspendiert die Zellen. Anschliessend wird ein 2000 ml Schüttelkolben, enthaltend 1000 ml Medium 2 (bestehend aus 10 Gew.-% Glucose, 1 Gew.-% Malzextrakt und 89 Gew.-% destilliertem Wasser), mit 10 ml der Suspension beimpft. Nach einer Inkubation von 3

Tagen unter Schütteln bei 30° wird ein 15 l Bioreaktor. enthaltend 10 l Medium 2. mit der Vorkultur beimpft. Nach 3 Tagen Inkubation (30°. 250 rpm) beginnt man portionenweise (0.78 g Stunde) Aethyl-α-1.3-trioxo-2-isoindolbutyrat in Pulverform zuzugeben (total 186 g). 240 Stunden nach Beginn der Zugabe wird der Prozess abgebrochen. nachdem das eingesetzte Ausgangsprodukt umgesetzt ist. Die Transformationsausbeute beträgt 88%. Das gebildete (R)-Aethyl-α-hydroxy-1,3-dioxo-2-isoindolbutyrat zeigt ein ee von 96%. Nach anschliessender Extraktion mit Aethylacetat und mehrfacher Kristallisation erhält man 155,4 g reines (R)-Aethyl-α-hydroxy-1,3-dioxo-2-isoindolbutyrat mit einer ee von 97,9%. Die Totalausbeute beträgt demnach 83,5%.

Beispiel 4

Die Hefe Candida parapsilosis NRRL Y-2315 wird wie in Beispiel 3 beschrieben mit Medium 1 und 2 angezüchtet und im Bioreaktor mit Medium 2 vorkultiviert. Nach 4 Tagen Inkubation (30°, 250 rpm) beginnt man portionenweise (0,3 g/Stunde) Isopropyl-α-1,3-dioxo-2-isoindolbutyrat in Pulverform zuzugeben (total 40 g). Nach 130 Stunden wird der Prozess abgebrochen. nachdem das eingesetzte Ausgangsprodukt umgesetzt ist. Das gebildete (R)-Isopropyl-α-hydroxy-1,3-dioxo-2-isoindolbutyrat zeigt ein ee von 98.5%. Nach anschliessender Extraktion mit Aethylacetat und mehrfacher Kristallisation erhält man 33,5 g reines (R)-Isopropyl-α-hydroxy-1,3-dioxo-2-isoindolbutyrat mit einer optischen Reinheit von 98,2%. Die Totalausbeute beträgt demnach 83,8%.

Beispiel 5

Die Hefe Torulopsis magnoliae NRRL Y-2024 wird auf einem Schrägagar mit Medium 1 (bestehend aus 1 Gew.-% Hefextrakt, 1 Gew.-% Pepton, 2 Gew.-% Glucose, 1,6 Gew.-% Agar und 94,4 Gew.-% destilliertem Wasser) gezüchtet. Nach 3 Tagen Inkubation bei 30° gibt man 9 ml 0,9%iger (Gew./Vol.) Kochsalzlösung zu und suspendiert die Zellen. Anschliessend wird ein 1000 ml-Schüttelkolben, enthaltend 200 ml Medium 2 (bestehend aus 10 Gew.-% Glucose, 1 Gew.-% Malzextrakt und 89 Gew.-% destilliertem Wasser), mit 2 ml der Suspension beimpft. Nach einer Inkubation von 3 Tagen unter Schütteln bei 30° beginnt man Aethyl-α-1,3-trioxo-2-isoindolbutyrat portionenweise in Form einer 16%igen Aethylacetatlösung zuzugeben (0,04 ml/Stunde). Nach 96 Stunden wird der Prozess abgebrochen, nachdem 600 mg des eingesetzten Ausgangsprodukts (entspricht 0,3% des Fermentationsvolumens) umgesetzt sind. Die Transformationsausbeute beträgt 86%. Das gebildete (S)-Aethyl-α-hydroxy-1,3-dioxo-2-isoindolbutyrat zeigt ein ee von 97,4%.

Beispiel 6

Die Hefe Torulopsis magnoliae NRRL Y-2024 wird wie in Beispiel 3 beschrieben mit Medium 1 und 2 angezüchtet. Nach einer Inkubation von 3 Tagen bei 30° beginnt man Isopropyl-α-1.3-trioxo-2-isoindolbutyrat portionenweise in Form einer 16%igen Aethylacetatlösung zuzugeben (0.2 ml/Stunde). Nach 220 Stunden wird der Prozess abgebrochen, nachdem 800 mg des eingesetzten Ausgangsprodukts (entspricht 0,4% des Fermentationsvolumens) umgesetzt sind. Die Transformationsausbeute beträgt 83%. Das gebildete (S)-Isopropyl-α-hydroxy-1,3-dioxo-2-isoindolbutyrat zeigt ein ee von 97%.

Beispiel 7

a) Man suspendiert 1004 g (3,44 Mol) (R)-Isopropyl-α-hydroxy-1,3-dioxo-2-isoindolbutyrat in 5,65 l 25%iger Salzsäure und heizt während 8,5 Stunden unter gleichzeitigem Abdestillieren von Isopropanol; man lässt dann über Nacht bei Raumtemperatur stehen, rührt das Gemisch während 2 Stunden im Eisbad, filtriert, wäscht den Filterrückstand mit 1,5 l Wasser und dampft die vereinigten Filtrate ein, wobei ein öliger Rückstand verbleibt. Man verrührt zweimal mit 200 ml Wasser, dampft jeweils ein, verrührt zweimal mit 500 ml Acetonitril, dampft wiederum jeweils ein, gibt dann 3 l Acetonitril zu und lässt über Nacht bei Raumtemperatur unter Rühren kristallisieren. Der Niederschlag wird abfiltriert, mit 1 l Acetonitril und 1,5 l Hexan gewaschen und bei 35° im Vakuum getrocknet. Man erhält (R)-4-Amino-2-hydroxybuttersäure-hydrochlorid vom Smp. 119-121°.

b) In einer Säule werden 4.5 kg Ionenaustauscherharz (Dowex 44) mit Wasser aufgeschlämmt. Hierauf behandelt man das Harz mit 20-30 l 2n Natronlauge, bis im Eluat keine Chloridionen mehr nachgewiesen werden können, und anschliessend wird mit 20-30 l ionenfreiem Wasser gewaschen, bis das Eluat neutral ist.

Man löst 750 g (R)-4-Amino-2-hydroxybuttersäure-hydrochlorid unter leichtem Erwärmen in 1 l Wasser, kühlt die entstandene klare Lösung auf Raumtemperatur, bringt sie auf die gemäss obigen Angaben vorbehandelte Säule und eluiert mit Wasser. Die ersten 500 ml Eluat werden verworfen. Die folgenden 10 l Eluat werden gesammelt und auf ein Volumen von ca. 2 l eingeengt; die erhaltene Lösung enthält noch Chloridionen. Man regeneriert die Säule wie oben beschrieben, bringt die erhaltene Lösung nochmals auf die Säule und eluiert erneut mit Wasser. Wiederum werden die ersten 500 ml Eluat verworfen und die folgenden 10 l Eluat gesammelt. Durch Eindampfen und Trocknen im Vakuum bei 50° erhält man einen weissen Rückstand, in welchem keine Chloridionen nachgewiesen werden können.

Man löst den erhaltenen Rückstand unter Erwärmen in 1,95 l Wasser und tropft innerhalb von 2 Stunden 7,8 l Aethanol zu, wobei Kristallisation einsetzt. Hierauf rührt man über Nacht bei Raumtemperatur und kühlt das Gemisch zur Vervollständigung der Kristallisation während 4 Stunden im Eisbad. Der Niederschlag wird abfiltriert, mit 2 l eines eiskalten Gemischs von Aethanol und Wasser (4:1) und dann mit 0,5 l Aethanol gewaschen und schliesslich im Hochvakuum bei 50° über Kaliumhydroxid getrocknet. Man erhält (R)-4-Amino-2-hydroxybuttersäure in Form eines weissen Produkts vom Smp. 208-210°.

## Ansprüche

1. Verfahren zur Herstellung von optisch aktiven 2-Hydroxybuttersäurederivaten der allgemeinen Formel

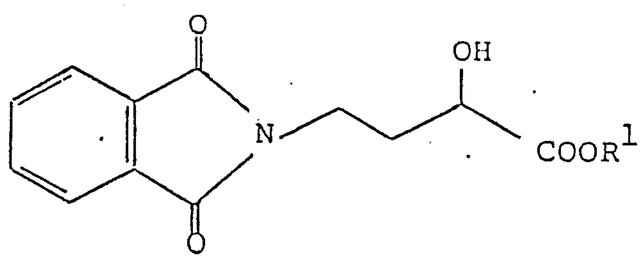

I

worin $R^1$ Wasserstoff oder einen üblichen Esterrest bedeutet, dadurch gekennzeichnet, dass man eine Verbindung der allgemeinen Formel

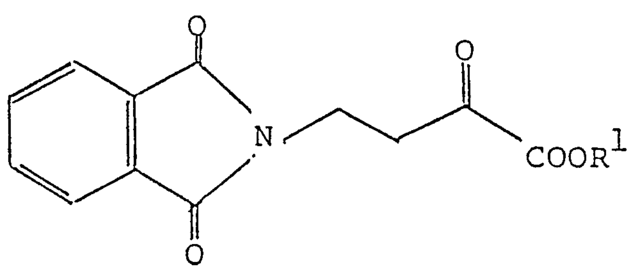

II

worin $R^1$ obige Bedeutung besitzt, fermentativ reduziert.

2. Verfahren nach Anspruch 1, dadurch gekennzeichnet, dass ein die Verbindung der Formel I erzeugender Mikroorganismus in einem wässrigen Nährmedium in Anwesenheit einer Verbindung der Formel II gezüchtet wird oder nach der Anzucht in Anwesenheit einer Verbindung der Formel II weiter inkubiert wird.

3. Verfahren nach Anspruch 1 oder 2, dadurch gekennzeichnet, dass man einen Mikroorganismus aus der Klasse der Actinomyceten, der Pilze oder der Hefen verwendet.

4. Verfahren nach Anspruch 3, dadurch gekennzeichnet, dass man einen Mikroorganismus der Gattung Actinoplanes, Rhodococcus, Beauveria, Byssochlamys, Choanephora, Glomerella, Candida, Hansenula, Rhodosporidium, Rhodotorula, Saccharomyces oder Torulopsis verwendet.

5. Verfahren nach Anspruch 4, dadurch gekennzeichnet, dass man einen Mikroorganismus der Species Actinoplanes sp., Rhodococcus rhodochrous, Beauveria bassiana, Byssochlamys fulva, Choanephora circinans, Glomerella rubicola, Candida albicans, Candida parapsilosis, Candida rugosa, Candida sorbophila, Candida tropicalis, Candida utilis, Hansenula anomala, Rhodosporidium toruloides, Rhodotorula minuta, Saccharomyces cerevisiae, Torulopsis magnoliae oder Torulopsis rotundata verwendet.

6. Verfahren nach Anspruch 5, dadurch gekennzeichnet, dass man als Mikroorganismus Candida parapsilosis DSM 3907, Torulopsis magnoliae DSM 3906 oder eine Mutante davon verwendet.

7. Verfahren nach einem der Ansprüche 1 bis 6, dadurch gekennzeichnet, dass $R^1$ Wasserstoff, Alkyl, Aryl, Aralkyl oder Alkylaryl bedeutet.

8. Verfahren nach Anspruch 7, dadurch gekennzeichnet, dass $R^1$ Wasserstoff, $C_1$-$C_6$-Alkyl, Phenyl, Benzyl oder Tolyl bedeutet.

9. Verfahren nach Anspruch 8, dadurch gekennzeichnet, dass $R^1$ Aethyl oder Isopropyl bedeutet.

10. Verfahren nach einem der Ansprüche 1 bis 9, dadurch gekennzeichnet, dass man die Reaktion bei einem pH-Wert von 3 bis 8 durchführt.

11. Verfahren nach einem der Ansprüche 1 bis 10, dadurch gekennzeichnet, dass man die Reaktion bei einer Temperatur von 20°C bis 35°C durchführt.

12. Verwendung der nach dem Verfahren gemäss einem der Ansprüche 1 bis 11 erhaltenen Verbindungen der Formel I zur Herstellung von optisch aktiver 4-Amino-2-hydroxybuttersäure, dadurch gekennzeichnet, dass man die Phthalimidogruppe und eine allfällige Estergruppe spaltet.